# FASCICULE DE BREVET EUROPEEN

(11) **EP 3 123 462 B1**
(45) Date de publication et mention de la délivrance du brevet: **07.10.2020**
(21) Numéro de dépôt: 15714874.3
(22) Date de dépôt: 18.03.2015
(51) Int. Cl.: G09G 3/34, G09G 3/20, A61N 1/05, A61F 2/14

(54) **PROCEDE DE COMMANDE D'AFFICHAGE ET DISPOSITIF POUR LA MISE EN OEUVRE DU PROCEDE**
ANZEIGESTEUERUNGSVERFAHREN UND VORRICHTUNG ZUR DURCHFÜHRUNG DIESES VERFAHRENS
DISPLAY CONTROL METHOD AND DEVICE FOR IMPLEMENTING SAID METHOD

(30) Priorité: 26.03.2014 FR 1452557
(43) Date de publication de la demande: 01.02.2017
(73) Titulaire: CNRS Centre National de la Recherche Scientifique, 75016 Paris (FR); Sorbonne Université, 75006 Paris (FR)
(72) Inventeur: BENOSMAN, Ryad, F-93500 Pantin (FR); CHENEGROS, Guillaume, F-78190 Trappes (FR); KIME, Sihem, F-75011 Paris (FR); IENG, Siohoi, F-93100 Montreuil (FR); SAHEL, José-Alain, 75013 Paris (FR)
(74) Mandataire: Plasseraud IP
(86) Numéro de dépôt international: PCT/FR2015/050654
(87) Numéro de publication internationale: WO 2015/145025

(56) Documents cités:
- WO-A1-2006/128315
- "Digital and Mixed Signal Oscilloscopes MSO/DPO70000 Series Datasheet", , 10 avril 2013 (2013-04-10), XP055137951, Extrait de l'Internet: URL:http://www.tek.com/sites/tek.com/files /media/media/resources/MSO-DPO70000-Oscill oscope-Datasheet-23.pdf [extrait le 2014-09-03]
- KWABENA A BOAHEN: "Point-to-Point Connectivity Between Neuromorphic Chips Using Address Events", IEEE TRANSACTIONS ON CIRCUITS AND SYSTEMS II: ANALOG AND DIGITALSIGNAL PROCESSING, INSTITUTE OF ELECTRICAL AND ELECTRONICS ENGINEERS INC, 345 EAST 47 STREET, NEW YORK, N.Y. 10017, USA, vol. 47, no. 5, 1 mai 2000 (2000-05-01), XP011013218, ISSN: 1057-7130
- S.R.Deiss R.J.Douglas A.M.Whatley: "A pulse coded communications infrastructure for neuromorphic systems" In: W. Maass and C.M. Bishop: "Pulsed natural networks", 31 décembre 1999 (1999-12-31), Cambridge, XP009179985, vol. 6, pages 157-178, le document en entier
- POSCH C ET AL: "A Microbolometer Asynchronous Dynamic Vision Sensor for LWIR", IEEE SENSORS JOURNAL, IEEE SERVICE CENTER, NEW YORK, NY, US, vol. 9, no. 6, 1 June 2009 (2009-06-01), pages 654-664, XP011256553, ISSN: 1530-437X
- Zhenjiang Ni: "Asynchronous Event Based Vision: Algorithms and Applications to Microrobotics", , 26 November 2013 (2013-11-26), XP055546230, Retrieved from the Internet: URL:https://tel.archives-ouvertes.fr/tel-0 0916995/document

## Description

La présente invention se rapporte aux procédés de commande d'affichage d'informations visuelles à partir d'une information asynchrone, et à des dispositifs pour la mise en œuvre de ces procédés de commande.

Les méthodes classiques de pilotage des dispositifs d'affichage comportant un écran sur lequel l'information va être projetée prévoient en général un mode de fonctionnement synchrone dans lequel l'affichage d'images sous forme d'une matrice de pixels est effectué sensiblement périodiquement. La période d'affichage détermine la dynamique d'affichage du dispositif, et résulte le plus souvent d'un compromis entre les limites matérielles du dispositif (dont celles relatives aux capacités de calcul de celui-ci), et la nécessité d'atteindre une résolution temporelle procurant un confort visuel maximum.

Cette période d'affichage correspond en outre à la période à laquelle l'affichage des pixels de la matrice est rafraîchi, étant donné que l'affichage est piloté de manière à afficher l'ensemble des pixels de la matrice simultanément ou quasiment simultanément. Par exemple, l'affichage d'une trame vidéo s'effectuera dans certains systèmes en affichant périodiquement une succession de sous-trames (des sous-trames dîtes « paires », correspondant à une sous-matrice comprenant les lignes de numéro d'ordre pair de la matrice de pixels correspondant à la trame, et des sous-trames dîtes « impaires », correspondant à une sous-matrice comprenant les lignes de numéro d'ordre impair de la matrice de pixels correspondant à la trame), de sorte que l'ensemble des pixels de chaque sous-trame sera affiché (ou allumé) périodiquement, les sous-trames étant affichées en alternance à une fréquence deux fois plus élevée que la fréquence d'affichage des trames.

La résolution temporelle qu'il est possible d'atteindre notamment pour des systèmes destinés au grand public est ainsi limitée par le fait que les méthodes d'affichage utilisées prévoient un affichage quasiment simultané d'un grand nombre de pixels de la matrice de pixels correspondant, sur l'écran d'affichage, à une fréquence elle-même limitée par les capacités de traitement de ces systèmes.

Par ailleurs, il semble qu'il soit quasiment impossible de transposer les méthodes d'affichage classiques dans le contexte de la commande des dispositifs d'aide à la vision. En effet, les implants ou les procédés de traitement optogénétiques requièrent des signaux de stimulation lumineuse d'intensité beaucoup plus élevée que celle de la lumière ambiante. Un implant d'aide à la vision placé sous l'œil, comprenant typiquement une électrode autour de laquelle sont disposées une à trois photodiodes, ne fonctionnera efficacement que si ces photodiodes reçoivent une lumière sept fois plus puissante que celle de la lumière ambiante, de manière à ce que les photodiodes puissent émettre un stimulus. De même, les traitements optogénétiques actuels ne sont pleinement efficaces que si l'œil traité reçoit des signaux lumineux ayant une longueur d'onde spécifique et une intensité lumineuse allant de deux à sept fois celle de la lumière ambiante. Les puissances lumineuses requises sont donc tellement élevées que l'utilisation des méthodes d'affichage classiques, à ces niveaux de puissance, provoquerait des lésions sur les organes visuels des utilisateurs.

Il existe ainsi un besoin pour des méthodes d'affichage, ne présentant pas les inconvénients des méthodes classiques exposés ci-dessus. En particulier, un premier besoin est de fournir des méthodes de commande d'affichage permettant d'atteindre des résolutions d'affichage plus élevées que celles résultant des compromis effectués avec des systèmes classiques. Un autre besoin est de fournir des méthodes de commande d'affichage qu'il soit possible d'utiliser pour des applications dans le domaine de l'aide à la vision.

Le document Zhenjiang Ni : « Asynchronous Event Based Vision : Algorithmes and Applications to Microrobotics », XP055546230, décrit un système de capture d'images sur évènements.

Selon un aspect, il est proposé un procédé de commande pour l'affichage d'images sous forme d'une matrice de pixels tel que revendiqué.

Dans un ou plusieurs modes de réalisation, le procédé comprend pour un pixel de la matrice : recevoir de l'information asynchrone représentant des évènements pour le pixel, commander une première activation du pixel à un instant d'activation déterminé par un premier évènement de l'information asynchrone, et commander au moins une deuxième activation du pixel pour répéter la première activation du pixel à des instants respectifs définis par une séquence de rafraichissement.

Les évènements concernant le pixel peuvent selon le mode de réalisation correspondre à des variations de lumière pour le pixel, à la détection d'une forme d'intérêt ou à la détection d'une primitive, et plus généralement à tout type d'information asynchrone pour le pixel.

Les procédés d'acquisition ou de synthèse d'une séquence d'images par trames présentent l'inconvénient de produire des données fortement redondantes du fait de l'échantillonnage dans le temps selon une fréquence d'horloge trame prédéfinie. Chaque trame représente un grand nombre de pixels d'une image si ce n'est une image entière, et inclut des informations relatives à des pixels pour lesquels l'information ne change pas d'une trame à l'autre, ce qui génère des redondances dans les données représentant la séquence d'images. Cette redondance ne peut être qu'en partie supprimée par un encodage de compression d'un signal vidéo classique. A l'inverse, l'utilisation pour chaque pixel de l'information asynchrone basée sur évènement (« event based ») permet d'obtenir une représentation très compacte d'une séquence d'images, du fait que l'information asynchrone représente des évènements. Dès lors, lorsque les évènements considérés correspondent par exemple à des variations de lumière pour le pixel, l'absence de variations de lumière pour un pixel se traduit par l'absence d'événement pour ce pixel, ce qui ne génère donc pas d'information redondante pour ce pixel dans la représentation de la séquence d'images.

L'activation d'un pixel à partir d'une information asynchrone peut être effectué en respectant - à une résolution temporelle près - le caractère asynchrone de la séquence d'évènements pour ce pixel, de manière à réaliser une activation du pixel qui est commandée sur évènement (« event driven »).

Dans un exemple de réalisation du procédé proposé, on répète la commande de première activation du pixel selon la séquence de rafraîchissement jusqu'à détermination d'un prochain instant d'activation du pixel par les évènements de l'information asynchrone.

Dans un mode de réalisation du procédé proposé, la réception de l'information asynchrone comprend la réception d'un signal portant l'information asynchrone, et la commande de première activation du pixel comprend la détection dans le signal d'une première information représentant le premier évènement et la commande d'activation sur détection de la première information représentant le premier événement.

De plus, la séquence de rafraîchissement peut définir des instants d'activation du pixel séparés par un intervalle de temps. Cet intervalle de temps entre une activation sur évènement et une activation de rafraîchissement, ou entre deux activations de rafraîchissement, peut par exemple être déterminé en fonction de la persistance rétinienne de l'œil humain. La persistance rétinienne de l'œil humain constitue un seuil limite qu'il est préférable de ne pas dépasser pour effectuer un affichage de rafraîchissement du pixel, au risque de détériorer le confort visuel de l'utilisateur. Par exemple, cet intervalle de temps sera choisi entre 40 ms et 800 ms, et de préférence entre 40 ms et 150 ms, afin d'éviter les effets de scintillement, sachant qu'un intervalle de temps plus long correspond à une fréquence de rafraîchissement moins élevée et une diminution du flux de commandes d'affichage et de calculs associés.

Dans un mode de réalisation du procédé proposé, les commandes d'activation sont délivrées sous la forme de stimulations électriques appliquées à une prothèse visuelle d'un dispositif d'aide à la vision.

La faible quantité de données représentant une séquence d'images d'un signal asynchrone de type AER permet d'augmenter l'intensité des signaux lumineux d'excitation des photorécepteurs d'une prothèse visuelle ou d'un organe visuel auquel un traitement optogénétique a été appliqué. En effet, le procédé proposé permet de ne considérer l'affichage d'un pixel que lorsqu'un évènement correspondant à ce pixel est détecté dans les données d'entrée représentant par exemple une séquence d'images, et de n'effectuer des affichages de rafraichissement qu'à une fréquence beaucoup plus faible que celle des procédés d'affichage classiques.

Dans un autre mode particulier de réalisation, les commandes d'activation sont configurées pour piloter l'affichage de pixels sur un écran d'affichage d'un dispositif.

Ainsi, le procédé proposé peut être utilisé pour la génération de commandes d'activation de pixels, en vue de l'affichage d'une séquence d'images sur l'écran d'un quelconque dispositif, comme par exemple un écran de téléviseur, de smartphone, de tablette numérique, de moniteur informatique, de GPS, etc.

Le procédé proposé est également applicable à tout système d'affichage par projection, par exemple sur un écran, qu'il soit physique ou virtuel.

Dans un mode de réalisation du procédé proposé, le rafraichissement de l'activation est effectué pour une pluralité de pixels de la matrice de pixels.

On peut en outre combiner l'affichage d'un pixel sur évènement qui reproduit le caractère asynchrone issu par exemple d'un signal asynchrone reçu pour affichage à un affichage de rafraichissement synchrone, c'est-à-dire périodique, qui peut prévoir un affichage de rafraichissement de tout ou partie de la matrice de pixels.

Il est préférable dans ce mode de réalisation avec affichage de rafraichissement synchrone de choisir une fréquence de rafraichissement beaucoup plus basse que celle des systèmes actuels, afin de mieux tirer parti de l'asynchronisme des affichages sur évènements pour chaque pixel.

Dans un mode de réalisation du procédé proposé, le signal asynchrone comporte un train d'impulsions binaires positionnées dans le temps en fonction des variations de lumière dans la scène pour le pixel. En variante, l'information représentative de l'événement peut comprendre une première impulsion positionnée dans le temps en fonction des variations de lumière dans la scène pour le pixel, et une deuxième impulsion positionnée dans le temps en fonction d'un niveau de gris pour l'affichage du pixel.

Dans un mode de réalisation du procédé proposé, correspondant à une situation dans laquelle un signal asynchrone porte de l'information représentative d'une pluralité d'évènements pour le pixel, le procédé proposé pourra en outre comprendre : détecter dans le signal asynchrone une information représentant un deuxième évènement pour le pixel, et, lorsque l'écart temporel entre les premier et deuxième évènements est inférieur à un seuil, ne pas générer de signal de commande pour l'affichage asynchrone du pixel sur détection de l'information représentant le deuxième événement. Le seuil, qui pourra être prédéterminé, pourra être choisi compris entre 1ms et 40ms, et par exemple fixé à une valeur de l'ordre de 30 ms.

Selon un autre aspect, il est proposé un dispositif de commande pour l'affichage d'images sous forme d'une matrice de pixels, comprenant une interface d'entrée configurée pour recevoir de l'information asynchrone représentant des évènements pour au moins un pixel de la matrice, et une unité de commande d'activation de pixel, couplé de manière opérationnelle à l'interface d'entrée, et configuré pour commander l'activation du pixel selon les différents modes de réalisation du procédé proposé.

Dans un mode de réalisation du dispositif proposé, les évènements considérés correspondent respectivement à des variations de lumière dans une scène pour le pixel.

Dans un mode de réalisation, le dispositif comprend en outre une interface de sortie, couplée de manière opérationnelle à l'unité de commande d'activation de pixel, pour délivrer des commandes d'activation du pixel.

Selon un autre aspect, il est proposé un afficheur d'images, comprenant une unité d'affichage procurant une matrice de pixels, et un dispositif de commande pour l'affichage d'images sous forme d'une matrice de pixels selon les différents modes de réalisation proposés pour commander l'unité d'affichage.

Selon un autre aspect, il est proposé un programme d'ordinateur, chargeable dans une mémoire associée à un processeur, et comprenant des portions de code pour la mise en œuvre des étapes du procédé proposé lors de l'exécution dudit programme par le processeur, ainsi qu'un ensemble de données représentant, par exemple par voie de compression ou d'encodage, ledit programme d'ordinateur.

D'autres particularités et avantages de la présente invention apparaîtront dans la description ci-après d'exemples de réalisation non limitatifs, en référence aux dessins annexés, dans lesquels :
La figure 1 est un schéma synoptique d'un dispositif de commande d'affichage adapté à la mise en œuvre du procédé proposé selon un mode de réalisation;
La figure 2a est un schéma synoptique d'un dispositif d'acquisition apte à générer un signal asynchrone adapté à la mise en œuvre du procédé proposé;
La figure 2b est un diagramme montrant un exemple de profil d'intensité lumineuse au niveau d'un pixel d'un capteur asynchrone ;
La figure 2c montre un exemple de signal délivré par le capteur asynchrone en réponse au profil intensité de la figure 2b ;
La figure 2d illustre la reconstruction du profil intensité à partir du signal de la figure 2c ;
Les figures 3a et 3b sont des diagrammes analogues à ceux des figures 2b et 2c illustrant un autre mode d'acquisition lumineuse par un capteur asynchrone ;
Les figures 4a et 4b sont des diagrammes illustrant des modes de réalisation du procédé proposé ;
Les figures 5a et 5b sont des diagrammes montrant une séquence temporelle d'évènements reçus dans un signal asynchrone pour la mise en œuvre du procédé proposé ;
Les figures 5c et 5d sont des diagrammes montrant une séquence temporelle de commandes d'affichage générées selon des modes de réalisation particuliers du procédé proposé ;
La figure 6 est un diagramme illustrant des intervalles de temps pris en compte dans des modes de réalisation du procédé proposé.
La figure 7 illustre la variation du nombre de pixels non rafraîchis en mode d'affichage (ou d'activation) asynchrone par rapport à un affichage (ou une activation) asynchrone en fonction du nombre de pixels contenus dans les zones de mouvement d'une séquence d'images.

Dans la description détaillée ci-après de modes de réalisation de l'invention, de nombreux détails spécifiques sont présentés pour apporter une compréhension plus complète. Néanmoins, l'homme du métier peut se rendre compte que des modes de réalisation peuvent être mis en pratique sans ces détails spécifiques. Dans d'autres cas, des caractéristiques bien connues ne sont pas décrites en détail pour éviter de compliquer inutilement la description.

L'invention sera décrite ci-après dans le cadre non limitatif d'une information asynchrone représentant, pour un pixel d'une matrice de pixels, des évènements correspondant à des variations de lumière pour le pixel. Les procédés et dispositifs proposés ne sont toutefois pas limités à ce mode de réalisation particulier, les évènements concernant le pixel pouvant selon le mode de réalisation correspondre à des variations de lumière pour le pixel, à la détection d'une forme d'intérêt ou à la détection d'une primitive, et plus généralement à tout type d'information asynchrone pour le pixel.

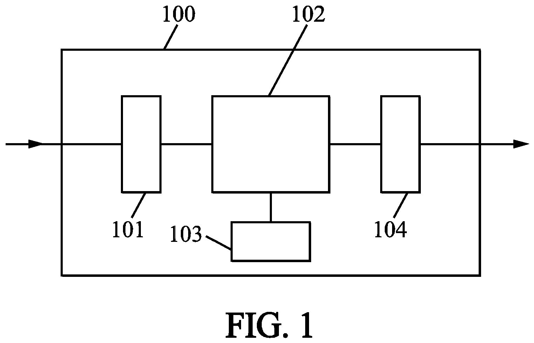

La figure 1 montre un système de commande d'affichage (100) comprenant une interface d'entrée (101) pour recevoir une information asynchrone, une unité de traitement de données (102), des moyens mémoire (103), et une interface de sortie (104) pour transmettre des signaux de commande d'affichage.

Dans un mode de réalisation du procédé proposé, l'information asynchrone reçue sur l'interface d'entrée (101) représente des évènements correspondant respectivement à des variations de lumière dans une scène pour un pixel. Elle correspond ainsi à une séquence d'images, chacune considérée sous la forme d'une matrice de pixels, un pixel étant un objet élémentaire d'une image. L'information asynchrone reçue sur l'interface (101) peut aussi représenter des évènements correspondant respectivement à des variations de lumière sur la séquence d'images pour un ensemble de pixels de la matrice, voire pour tous les pixels de la matrice.

Cette information peut être générée par différents moyens. Par exemple, elle peut être portée par un signal asynchrone produit par un capteur de vision asynchrone et reçu sur l'interface d'entrée (101). Elle peut aussi résulter de l'encodage d'une séquence d'images de synthèse produisant un ensemble de données reçu sur l'interface (101).

En outre, le procédé proposé n'est pas limité à un format particulier d'information asynchrone. D'une manière générale, l'information asynchrone représente des évènements relatifs aux pixels de la matrice. Dans un mode de réalisation particulier, l'information asynchrone indique, ou signale, des évènements relatifs à un ou plusieurs pixels. Elle peut par exemple être contenue dans des données qui identifient des évènements avec leurs caractéristiques respectives.

On considère dans la suite le cadre non limitatif d'une information asynchrone portée par un signal reçu sur l'interface (101) sous forme éventuellement codée pour sa transmission et/ou son stockage.

Dans ce mode de réalisation, le signal asynchrone reçu sur l'interface d'entrée (101) porte de l'information représentative d'évènements temporels correspondants à des variations de lumière dans une scène. Dans ce cas de figure, l'interface d'entrée 101 peut être configurée pour recevoir un signal produit par un sous-système de capture d'images asynchrone basé sur évènements. Ce sous-système incorpore typiquement un capteur de vision asynchrone basé sur évènements.

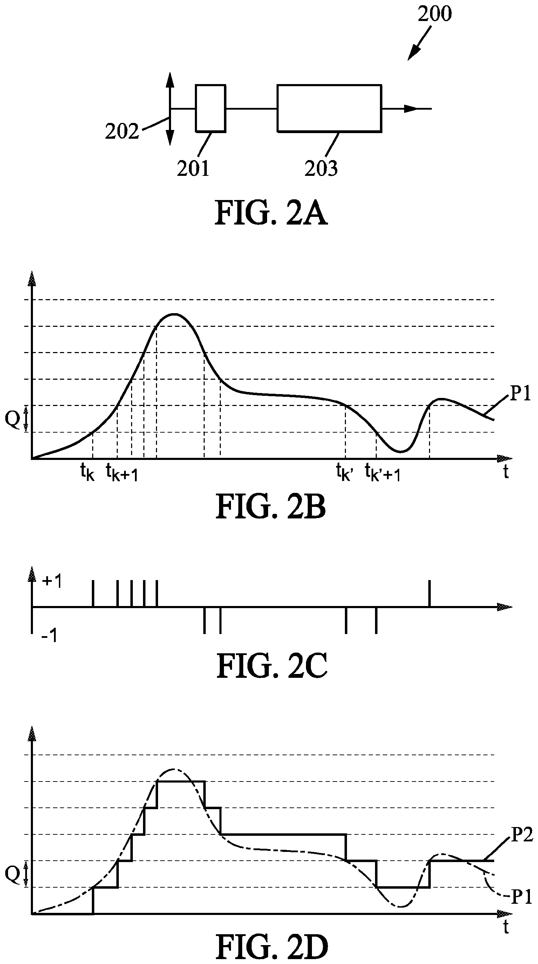

La figure 2a montre un dispositif d'acquisition de la lumière (200) comprenant un capteur de vision asynchrone basé sur évènements (201) placé en regard d'une scène et recevant le flux lumineux de la scène à travers une optique d'acquisition (202). Le capteur (201) peut comporter un groupement d'éléments photosensibles organisés en une matrice de pixels, de sorte que chaque pixel de la matrice corresponde à un élément photosensible du capteur. Pour chaque pixel de la matrice, le dispositif (200) engendre une séquence de signal asynchrone basé sur évènement à partir des variations de lumière ressenties par le pixel dans la scène apparaissant dans le champ de vision du dispositif (200). Chaque pixel correspondant à un élément photosensible, produit ainsi des évènements temporels correspondants respectivement à des variations de lumière dans la scène.

Le capteur 201 ne produit donc pas des trames vidéo constituées par la matrice de pixels correspondant aux éléments photosensibles du capteur à une fréquence d'échantillonnage prédéterminée. Il réagit pour chaque pixel de la matrice à des évènements correspondant à des variations de lumière pour le pixel. A l'inverse, il ne produit pas d'information pour un pixel si aucun évènement ne se produit pour ce pixel. Il n'effectue en particulier pas de capture systématique d'intensité lumineuse des pixels de la matrice. Ainsi, les évènements auxquels il réagit sont asynchrones, et ne dépendent pas d'une fréquence d'acquisition de trames vidéo. Cela permet de diminuer fortement, si ce n'est supprimer, les redondances crées par l'acquisition de trames vidéo à un rythme déterminé ne tenant pas compte de l'absence de changement de l'information portée par un pixel d'une trame à l'autre.

Un calculateur (203) traite l'information issue du capteur (201) et représentative des évènements produits de manière asynchrone par les différents pixels, pour générer un signal asynchrone portant cette information.

Un exemple de principe d'acquisition par ce capteur asynchrone est illustré par les figures 2b-2d. Selon cet exemple, l'information consiste en une succession d'instants, notés *tₖ* (*k* = 0,1,2,...) auxquels un seuil d'activation Q est atteint. Le capteur 201 est donc muni d'un détecteur de variations qui, pour chaque pixel, mesure et enregistre l'intensité lumineuse du pixel lorsque cette intensité a varié au-delà d'un seuil Q.

La figure 2b montre un exemple de profil d'intensité lumineuse P1 vue par un pixel de la matrice du capteur de vision asynchrone. Chaque fois que cette intensité augmente d'une quantité égale au seuil d'activation Q en comparaison de ce qu'elle était à l'instant tₖ, un nouvel évènement est identifié et une raie positive (niveau +1 sur la figure 2c) est émise correspondant à l'instant de dépassement du seuil différentiel Q, noté tₖ₊₁. Symétriquement, chaque fois que l'intensité du pixel diminue de la quantité Q en comparaison de ce qu'elle était à l'instant t_{k'}, un nouvel évènement est identifié et une raie négative (niveau -1 sur la figure 2c) est émise correspondant à l'instant de dépassement du seuil différentiel Q, noté t_{k'+1}.

Le signal asynchrone pour le pixel consiste alors en une succession d'impulsions ou raies, positives ou négatives, positionnées dans le temps aux instants tₖ dépendant du profil lumineux pour le pixel. Ces raies peuvent être représentées mathématiquement par des pics de Dirac positifs ou négatifs et caractérisées chacune par un instant d'émission tₖ et un bit de signe. La sortie du capteur 201 est ainsi sous la forme d'une représentation adresse-événement AER. L'information produite par le capteur, correspondant à un évènement pour un pixel, comprend une première information relative à un instant d'occurrence de l'évènement, et une deuxième information relative à une caractéristique lumineuse pour le pixel à cet instant.

La figure 2d montre le profil d'intensité P2 qu'on est capable de reconstruire comme une approximation du profil P1 par intégration dans le temps du signal asynchrone de la figure 2c.

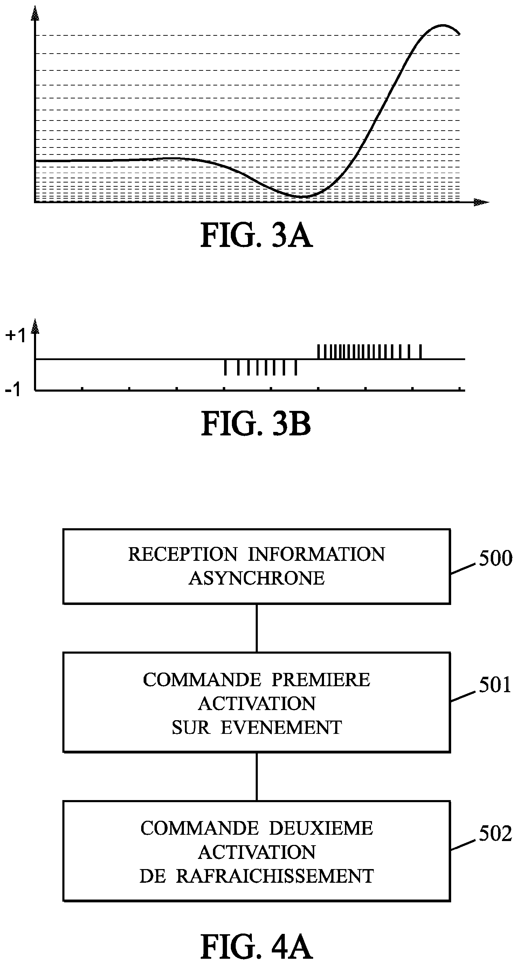

Le seuil d'activation Q peut être fixe, comme dans le cas des figures 2b-d, ou adaptatif en fonction de l'intensité lumineuse, comme dans le cas des figures 3a-b. Par exemple, le seuil ±Q peut être comparé aux variations du logarithme de l'intensité lumineuse pour la génération d'un événement ±1. Le choix de l'échelle logarithmique pour la mesure de l'intensité lumineuse présente ainsi deux avantages : d'une part, il est mieux adapté à la physiologie de l'œil du fait que la sensibilité à la lumière de la rétine est logarithmique, et d'autre part, comme indiqué ci-dessus, le seuil défini dans l'échelle logarithmique peut être adaptatif.

À titre d'exemple, le capteur 201 peut être du genre décrit dans "A 128x128 120 dB 15 µs Latency Asynchronous Temporal Contrast Vision Sensor", P. Lichtsteiner, et al., IEEE Journal of Solid-State Circuits, Vol. 43, No. 2, février 2008, pp. 566-576, ou dans la demande de brevet US 2008/0135731 A1, qui décrivent une première génération de capteurs asynchrones, usuellement désignés sous l'acronyme DVS, pour « Dynamic Vision Sensor ».

La dynamique de la rétine (durée minimum entre les potentiels d'action) de l'ordre de quelques millisecondes peut être convenablement reproduite avec un capteur de ce type. La performance en dynamique est en tout cas largement supérieure à celle qu'on peut atteindre avec une caméra vidéo classique ayant une fréquence d'échantillonnage réaliste. Par exemple, un capteur de ce type permet d'atteindre des résolutions temporelles de l'ordre de la microseconde avec une gamme de luminance supérieure à 120 dB, ce qui est très supérieur à une caméra standard CMOS/CCD qui possède typiquement une gamme de luminance de 60-70 dB.

Il est à noter que la forme du signal asynchrone délivré pour un pixel par le capteur 201, qui constitue le signal d'entrée du calculateur 203, peut être différente d'une succession de pics de Dirac, les événements représentés pouvant avoir une largeur temporelle ou une amplitude ou une forme d'onde quelconque dans ce signal asynchrone basé sur événement.

Chaque pixel du capteur de position p = (x ; y), délivre ainsi une suite d'impulsions binaires, que l'on peut modéliser par des évènements ON ou OFF engendrés de manière asynchrone à des instants respectifs *tₖ*. Le calculateur 203 est configuré pour générer un signal asynchrone portant l'ensemble des évènements pour un ou plusieurs pixels de la matrice. L'information correspondant à un événement comprend une information de position du pixel pour lequel l'événement s'est produit (par exemple le couple (x ; y) des numéros de ligne et de colonne dans la matrice de pixels), une information du temps d'occurrence de l'événement (par exemple une valeur discrète de temps par rapport à une référence), et une information de type d'événement (par exemple un bit pour coder des évènements de deux types).

En référence à la figure 1, le signal asynchrone reçu en entrée de l'interface 101 peut ainsi porter de l'information représentative d'un flux d'évènements, chacun défini par un quadruplet *e*(*x* ; *y* ; *t* ; *ON*/*OFF*) donnant la position p = (x ; y) du pixel auquel l'événement est associé, l'instant t auquel l'événement a été détecté, et le type (ON ou OFF) de l'évènement.

Dans un autre mode particulier de réalisation, le signal asynchrone reçu en entrée de l'interface 101 porte de l'information représentative d'un flux d'évènements où chaque évènement est défini par un quadruplet *e*(*x; y; t* ; *g*) donnant la position p = (x ; y) du pixel auquel l'événement est associé, l'instant t auquel l'événement a été détecté, et un niveau de gris g associé à l'évènement.

L'article de Posch, C., Matolin, D., et Wohlgenannt, R. (2011) intitulé "A qvga 143 db dynamic range frame-free pwm image sensor with lossless pixel-level video compression and time-domain cds", et publié dans le IEEE Journal of Solid-State Circuits, 46, pages 259 -275. doi:10.1109/JSSC.2010.2085952, fournit une description d'exemples d'évènements codés par des niveaux de gris.

L'information asynchrone pour chaque pixel consiste là-encore en une succession d'impulsions ou raies positionnées dans le temps aux instants *tₖ* dépendant du profil lumineux pour le pixel. Chaque évènement peut par exemple correspondre à deux impulsions successives, la première indiquant l'instant de l'événement et la seconde permettant de déterminer un niveau de gris pour le pixel en fonction de l'écart temporel entre les deux impulsions. L'information correspondant à un évènement pour un pixel comprend ainsi une première information relative à un instant d'occurrence de l'évènement, et une deuxième information relative à une caractéristique lumineuse (niveau de gris) pour le pixel à cet instant.

Cette nouvelle génération de capteurs capables de générer un niveau de gris pour chaque évènement est parfois désignée sous l'acronyme ATIS, pour « Asynchronous, Time-Based Image Sensor ». Le sous-système de capture et le capteur ATIS qu'il incorpore peuvent être par exemple du type de celui décrit dans l'article de C. Posch et al., intitulé « An Asynchronous Time-based Image Sensor » (IEEE International Symposium on Circuits and Systems, 2008, pages 2130-2133), ou bien du type de celui décrit dans l'article de C. Posch et al., intitulé « A QVGA 143 dB dynamic range frame-free PWM image sensor with lossless pixel-level video compression and time-domain CDS » (46(1) :259275, 2011).

En référence à la figure 1, l'unité de traitement des données 102 peut être un ordinateur, un réseau d'ordinateurs, ou un autre appareil comportant un processeur, éventuellement couplé de manière opérationnelle à une mémoire 103, et pouvant inclure en outre une unité de stockage de données, et d'autres éléments matériels associés comme une interface de réseau et un lecteur de support pour lire un support de stockage amovible et écrire sur un tel support (non représentés sur la figure). Le support de stockage amovible peut être, par exemple, un disque compact (CD), un disque vidéo/polyvalent numérique (DVD), un disque flash, une clé USB, etc. En fonction du mode de réalisation, la mémoire 103, l'unité de stockage de données ou le support de stockage amovible contient des instructions qui, lorsqu'elles sont exécutées par l'unité de traitement des données 102, amènent cette unité 102 à effectuer ou contrôler les parties interface d'entrée 101 et/ou de sortie 104 et/ou traitement de données des exemples de mise en œuvre du procédé proposé décrits dans les présentes. L'unité de traitement 102 peut être un composant implémentant un processeur ou une unité de calcul pour la génération de commandes d'affichage selon le procédé proposé et le contrôle des interfaces d'entrée 101 et de sortie 104 du système 100.

En outre, le système de commande d'affichage 100 peut être mis en œuvre sous forme logicielle, comme décrit ci-dessus, ou sous forme matérielle, comme un circuit intégré spécifique application (ASIC), ou sous forme d'une combinaison d'éléments matériels et logiciels, comme par exemple un programme logiciel destiné à être chargé et exécuté sur un composant de type FPGA (Field Programmable Gate Array).

Le système de commande 100 génère des commandes d'affichage dont le format est adapté au système d'affichage auquel ces commandes sont transmises par le biais de l'interface de sortie 104. Le procédé proposé est applicable à la commande de tous types de système d'affichage, tel que des systèmes d'affichage sur écran, des systèmes de projection, ou des dispositifs d'aide à la vision.

L'interface de sortie 104 pourra donc comprendre un module pilote (non représenté sur la figure 1) permettant d'adapter les signaux en sortie du système 100 à l'interface d'entrée du système d'affichage.

Par exemple, dans le cas où le système d'affichage est une prothèse installée sur la rétine, le module pilote de l'interface 104 convertira les signaux de commandes en des potentiels électriques analogiques qui seront transmis par le biais de l'interface 104 aux électrodes de la prothèse.

Dans un mode particulier de réalisation du procédé proposé, l'interface de sortie 104 est configurée pour générer un signal de sortie de format adapté pour piloter un projecteur vidéo utilisant la technologie des matrices de micro-miroirs (en anglais DMD, pour « Digital Micromirror Device »). Ce type de projecteur est parfois désigné sous l'acronyme DLP, pour « Digital Light Processing », et fonctionne avec une source de lumière qui vient illuminer une matrice de micro-miroirs qui vibrent en fonction de la quantité de lumière à réfléchir. Les vibrations de chaque miroir s'effectuent autour de deux positions correspondant respectivement à des angles d'inclinaison autour d'un axes, l'une dans laquelle la lumière est réfléchie par le miroir vers une optique de sortie, et l'autre dans laquelle la lumière est réfléchie par le miroir vers une surface absorbante et n'est donc pas projetée. Chaque miroir de la matrice DMD projette de la lumière pour un pixel sur un écran d'affichage.

Certains projecteurs DLP sont en outre capables d'illuminer des pixels avec différents niveaux de gris, et peuvent donc recevoir des commandes d'affichage pour chaque pixel comprenant une information relative à un niveau de gris avec lequel le pixel doit être affiché. On pourra par exemple utiliser un projecteur DLP capable de gérer 1024 niveaux de gris, et lui fournir des commandes d'affichage selon le procédé proposé dans lesquelles le niveau de gris d'illumination d'un pixel est codé sur 10 bits.

Le projecteur DLP piloté en utilisant le procédé proposé pourra par exemple être du type de celui monté sur les plateformes d'évaluation « DLP Lightcrafter ». Ce type de dispositif peut par exemple comprendre un composant DMD de taille 4,6mm x 5.2mm, et supporter une résolution d'affichage, c'est-à-dire une taille de l'image affichée en pixels, de 608 x 684 pixels (correspondant au standard WVGA) et une résolution temporelle allant jusqu'à 1440 Hz.

Ainsi, dans le cas où le système d'affichage est un projecteur DLP, le module pilote de l'interface 104 convertit les signaux de commandes pour interfacer le système de commande d'affichage 100 avec une entrée du projecteur DLP.

En référence aux figures 1 et 4a, le dispositif de commande d'affichage 100 pilote indépendamment chaque pixel d'une matrice de pixels pour l'activation de ce pixel. Le dispositif 100 reçoit (500) par le biais de l'interface d'entrée 101 de l'information asynchrone représentative d'évènements correspondants à des variations de lumière pour le pixel.

Par exemple, pour un pixel de position (xₒ, yₒ) dans la matrice de pixels (pixel positionné sur la ligne d'indice xₒ et sur la colonne d'indice yₒ dans une matrice MxN, avec *x* ∈ {0, *...,M* - 1} et *y* ∈ {0, *...,N -* 1}, l'information reçue comprendra de l'information asynchrone pour le pixel de position (xₒ, yₒ).

L'information asynchrone est traitée par l'unité de traitement de données 102 pour identifier un évènement pour le pixel afin de commander (501) une première activation du pixel à un instant d'activation déterminé par l'événement identifié.

Dans un mode de réalisation particulier, l'identification d'un évènement pourra viser des évènements caractérisés par une première information indiquant un instant de survenance de l'évènement, et une deuxième information relative à une caractéristique lumineuse pour le pixel à un instant correspondant. Par exemple, l'identification d'un évènement pourra comprendre la détection de deux pics ou impulsions dans un signal portant l'information asynchrone, le premier indiquant un instant de survenance de l'événement et le deuxième un niveau de gris caractéristique de l'événement pour le pixel.

Dans le mode de réalisation dans lequel les évènements sont caractérisés par l'instant de survenance d'une variation d'intensité lumineuse au-delà d'un seuil et le sens de cette variation, la commande d'activation pourra comprendre un niveau d'illumination du pixel déterminé en tenant compte de la variation détectée appliquée au niveau d'illumination de la précédente commande d'affichage du pixel.

Dans le mode de réalisation dans lequel les évènements sont caractérisés par l'instant de survenance d'une variation d'intensité lumineuse au-delà d'un seuil et un niveau de gris associé à cette variation, la commande d'activation pourra comprendre un niveau d'illumination du pixel correspondant au niveau de gris déterminé suite à la détection de l'évènement.

Cette première activation du pixel est donc commandée sur identification d'un évènement pour un pixel en utilisant l'information asynchrone reçue en entrée du système de commande d'affichage 100. Dans un mode particulier de réalisation, l'activation est pilotée dès qu'un évènement est identifié pour un pixel, moyennant le temps de traitement nécessaire pour le système 100 pour le traitement des informations relatives à l'évènement. En variante, le système 100 pourra maintenir une référence de temps d'activation, sur la base de laquelle les activations des pixels seront pilotées à des instants correspondant respectivement aux évènements identifiés pour chaque pixel. Comme décrit précédemment, chaque évènement peut être caractérisé par un instant d'occurrence et une ou plusieurs valeurs correspondant à des informations lumineuses respectives (intensité lumineuse, niveau de gris, couleur, etc.).

Une deuxième activation du pixel est commandée (502) suite à la première activation pour répéter celle-ci à des instants respectifs définis par une séquence de rafraichissement. La première activation est donc suivie d'une ou de plusieurs activations destinées à rafraîchir l'activation commandée sur identification d'un événement.

Dans un mode particulier de réalisation du procédé proposé, la séquence de rafraîchissement définit des instants d'activation du pixel séparés par un intervalle de temps. Cet intervalle de temps peut être commun à l'ensemble des pixels de la matrice ou bien défini pour chaque pixel ou pour différents sous-ensembles de pixels. Il peut notamment être déterminé en fonction de la persistance rétinienne de l'œil humain. Cela permet de choisir des valeurs d'intervalle de rafraîchissement suffisamment grandes pour éviter l'affichage d'informations redondantes à une fréquence élevée au détriment de l'efficacité du système, tout en tenant compte de la durée de la persistance rétinienne de l'affichage précédent. Par exemple, l'intervalle de temps de rafraîchissement peut être choisi entre 40 ms et 150 ms, sachant que plus la valeur choisie sera élevée plus le système de commande d'activation de pixels gagnera en efficacité en évitant d'autant plus les activations de redondance.

Une deuxième activation du pixel est donc commandée pour effectuer un rafraîchissement de l'activation du pixel pendant l'intervalle de temps ainsi déterminé et courant à partir de l'activation du pixel suite à la précédente commande d'activation.

Cette précédente commande d'activation peut être une commande d'activation sur identification d'événement telle que décrit ci-dessus, ou bien une commande d'activation de rafraîchissement dans le cas par exemple où aucune activation sur identification d'événement n'a été commandée pendant une durée correspondant à l'intervalle de temps de rafraîchissement courant depuis la précédente activation du pixel.

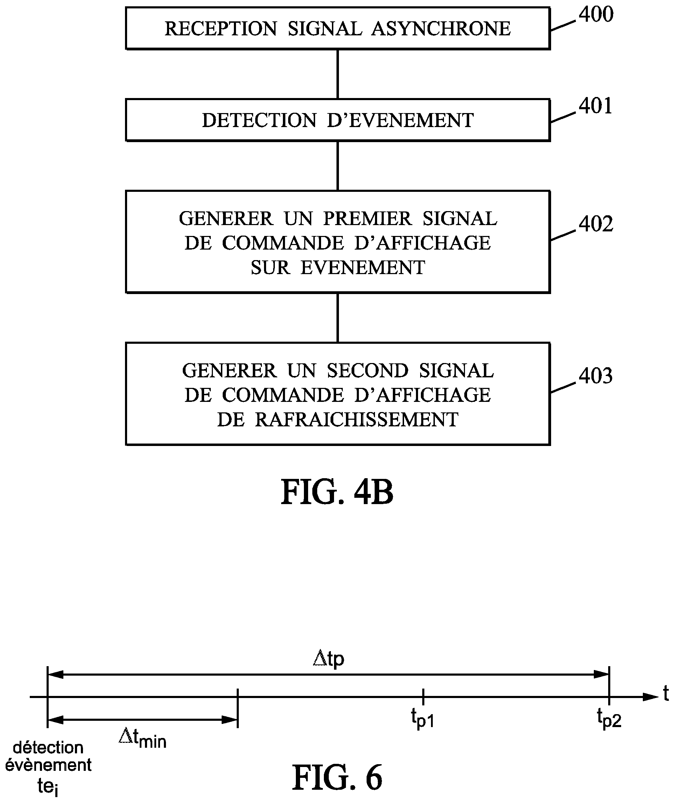

La figure 4b illustre un mode de réalisation particulier du procédé proposé, dans lequel le dispositif 100 de la figure 1 reçoit un signal portant de l'information asynchrone, traite ce signal pour détecter des évènements, puis génère des commandes d'activation de pixels pour l'illumination du pixel, par exemple par projection sur un écran d'affichage.

En référence aux figures 1 et 4b, le dispositif de commande d'affichage 100 pilote indépendamment chaque pixel d'une matrice de pixels pour l'activation de ce pixel. Le dispositif 100 reçoit (400) par le biais de l'interface d'entrée 101 un signal asynchrone portant de l'information représentative d'évènements correspondants à des variations de lumière pour le pixel.

Par exemple, pour un pixel de position (xₒ, yₒ) dans la matrice de pixels (pixel positionné sur la ligne d'indice xₒ et sur la colonne d'indice yₒ dans une matrice MxN, avec *x* ∈ {0, *..., M* -1} et *y* ∈ {0, *..., N* - 1}, le signal asynchrone reçu comprendra une séquence de signal pour le pixel de position (xₒ, yₒ).

Le signal asynchrone est traité par l'unité de traitement de données 102 pour détecter (401) une information représentant un évènement pour le pixel.

Dans un mode de réalisation, la détection d'une information représentant un évènement pourra viser des évènements caractérisés par l'instant de survenance d'une variation d'intensité lumineuse au-delà d'un seuil (qui pourra être, dans un mode de réalisation, spécifique au pixel) et le sens de cette variation. Par exemple, la détection pourra comprendre la détection d'une impulsion dont le positionnement par rapport à une référence de temps correspond à l'instant de survenance d'une variation d'intensité lumineuse au-delà d'un seuil et dont le type (par exemple ON ou OFF, ou bien +1 ou -1) correspondra au sens de la variation lumineuse.

Dans un autre mode de réalisation du procédé proposé, la détection d'une information représentant un évènement pourra viser des évènements caractérisés par l'instant de survenance d'une variation d'intensité lumineuse au-delà d'un seuil (qui pourra être, dans un mode de réalisation, spécifique au pixel) et un niveau de gris associé à cette variation. Par exemple, la détection pourra comprendre la détection d'une première impulsion dont le positionnement par rapport à une référence de temps correspond à l'instant de survenance d'une variation d'intensité lumineuse au-delà d'un seuil et la détection d'une deuxième impulsion dont l'écart temporel avec la première impulsion permettra de déduire un niveau de gris pour le pixel.

L'information représentant un évènement pour le pixel est traitée par l'unité de traitement de données 102 pour générer (402) un premier signal de commande d'affichage du pixel.

Le caractère asynchrone du signal d'entrée du dispositif de commande d'affichage 100 sera d'autant mieux respecté que le temps de génération et de transmission d'une commande d'affichage du pixel à partir du moment où un évènement est détecté pour le pixel sera court. Un traitement temps-réel sera ainsi préféré afin d'obtenir un affichage quasiment instantané des évènements détectés pour chaque pixel.

La première commande d'affichage, générée sur détection d'un évènement pour un pixel, est transmise au système d'affichage auquel le système de commande d'affichage 100 est interfacé par le biais de l'interface de sortie 104.

Dans les modes de réalisation utilisant un système d'affichage sur écran, l'affichage sur écran pourra être effectué suite à la réception de la commande d'affichage avec un retard correspondant au temps de traitement de la commande et à la résolution temporelle du système d'affichage.

Par exemple, comme indiqué ci-dessus, un projecteur DLP pourra être utilisé pour recevoir des commandes d'affichage générées selon le procédé proposé, dans une configuration où sa résolution temporelle est proche de 1440 Hz. Cette résolution temporelle de 1440 Hz correspond à une possibilité d'affichage toutes les 0,69 ms. Un projecteur DLP pourra ainsi projeter de la lumière correspondant à l'illumination d'un pixel d'une image au plus tard 0,69 ms après avoir reçu et traité une commande d'affichage générée selon le procédé proposé.

Ce premier affichage de pixel, effectué sur détection d'un évènement pour le pixel, est suivi d'un deuxième affichage destiné à rafraîchir le précédent affichage sur détection d'évènement. Ce deuxième affichage donne lieu à la génération (403) d'une deuxième commande d'affichage. Cette deuxième commande d'affichage est générée de manière à ce que l'affichage qui en découle permette le rafraîchissement de l'affichage précédemment effectué pour le pixel. Pour ce faire, on détermine un intervalle de temps de rafraichissement dont la durée définit l'écart temporel maximum entre deux affichages pour un pixel.

Cet intervalle de temps peut être commun à l'ensemble des pixels de la matrice ou bien défini pour chaque pixel ou pour différents sous-ensembles de pixels.

Dans un mode de réalisation du procédé particulier, il est déterminé en fonction de la persistance rétinienne de l'œil humain. Cela permet de choisir des valeurs d'intervalle de rafraîchissement suffisamment grandes pour éviter l'affichage d'informations redondantes à une fréquence élevée au détriment de l'efficacité du système, tout en tenant compte de la durée de la persistance rétinienne de l'affichage précédent.

Par exemple, l'intervalle de temps de rafraîchissement peut être choisi entre40 ms et 150ms, sachant que plus la valeur choisie sera élevée plus le système d'affichage gagnera en efficacité en évitant d'autant plus les affichages de redondance.

Un deuxième signal de commande d'affichage est donc généré (403) pour effectuer un rafraîchissement de l'affichage du pixel pendant l'intervalle de temps ainsi déterminé et courant à partir de l'affichage du pixel suite à la précédente commande d'affichage.

Cette précédente commande d'affichage peut être une commande d'affichage sur détection d'événement telle que décrit ci-dessus, ou bien une commande d'affichage de rafraîchissement dans le cas par exemple où aucune commande d'affichage sur détection d'événement n'a été générée pendant une durée correspondant à l'intervalle de temps de rafraîchissement courant depuis le précédent affichage.

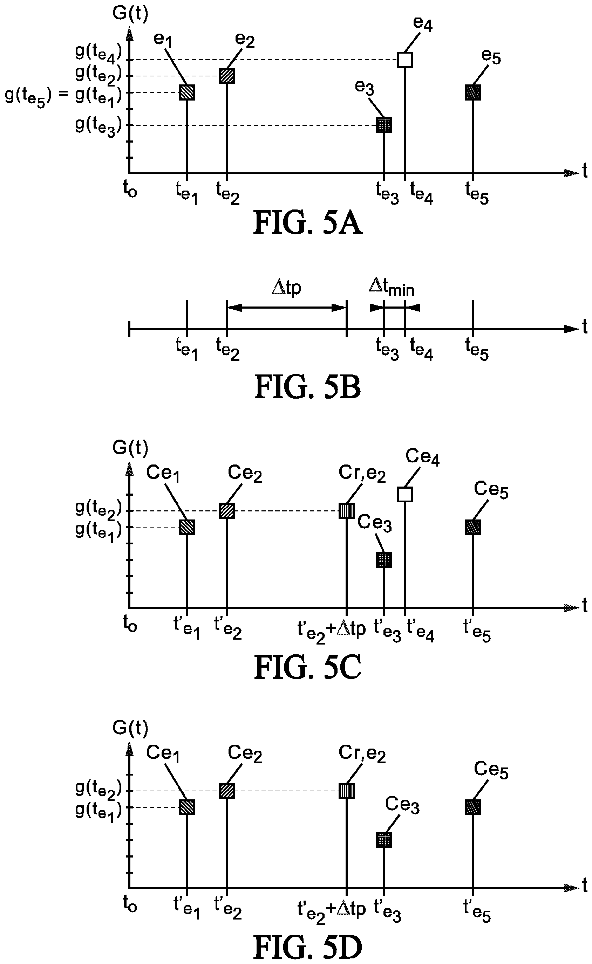

Les figures 5a et 5b illustrent une séquence de signal asynchrone pour un pixel et les commandes d'affichage correspondantes générées selon une mise en œuvre du procédé proposé.

La figure 5a illustre l'information portée par un échantillon de signal asynchrone dans lequel les évènements pour un pixel sont représentés sur un axe des temps par des pics de Dirac d'amplitude G(t) correspondant à un niveau de gris. Cinq évènements e₁, e₂, e₃, e₄, et e₅ sont représentés sur la figure 5a, positionnés respectivement aux instants te1, te2, te3, te4 et te5 par rapport à une référence de temps t0, avec te₁ < te₂ < te₃ < te₄ < te₅. Chacun des 5 évènements porte une information de niveau de gris pour le pixel considéré notée g(t=teᵢ), avec i = {1, 2, 3, 4, 5}

Ces valeurs de niveau de gris pourront par exemple résulter de la quantification d'une valeur de niveau de gris sur 2*^{n_{quant}}* niveaux et être codés sur n_{quant} bits.

La figure 5b montre une vue simplifiée de la séquence d'évènements illustrée par la figure 5a sur laquelle les évènements sont représentés par des pics de Dirac d'amplitude constante. L'intervalle de temps Δ*tₚ* est défini en fonction de la persistance rétinienne de l'œil et de manière à correspondre à un intervalle de temps maximum séparant deux affichages consécutifs d'un même pixel. Sur l'exemple illustré sur la figure 5b, les écarts temporels séparant deux évènements consécutifs n'excèdent pas la quantité Δ*tₚ*, à l'exception de l'écart entre les évènements e₂ et e₃ te₃ - te₂.

Dans l'exemple d'une séquence d'évènements illustrée sur les figures 5a et 5b, on pourra générer une commande d'affichage du pixel suite à la détection de l'évènement e₁ pour un affichage avec les caractéristiques (typiquement un niveau de gris) portées par le signal asynchrone traité pour l'évènement e₁. Il en est de même des évènements e₂, e₃, e₄, et e₅ dont les détections, dans un mode de réalisation, pourront chacune donner lieu à la génération d'une commande d'affichage avec des caractéristiques respectives.

Dans un mode particulier de réalisation du procédé proposé, une commande d'affichage sera en outre générée pour effectuer un affichage de rafraîchissement dans un intervalle de temps suite à l'affichage du pixel sur détection de l'évènement e₂. Cette commande d'affichage pourra par exemple être générée si aucun évènement n'a été détecté dans l'intervalle de temps [te₂ ; te₂+Δ*t*_{*p*]} de durée Δ*tₚ* à partir de l'instant te₂.

En variante elle pourra être générée à tout instant pendant la durée de l'intervalle de temps [te₂ ; te₂+Δ*tₚ*] de durée Δ*tₚ* à partir de l'instant te₂.

La figure 5c montre un exemple de séquence de commandes d'affichage générées selon un mode de réalisation du procédé proposé appliqué à la séquence d'évènements de la figure 5a. Les commandes d'affichage sont représentées sur la figure par des pics de Dirac d'amplitude variable. Elle montre des commandes d'affichages Ce₁, Ce₂, Ce₃, Ce₄ et Ce₅ correspondant respectivement aux évènements e₁, e₂, e₃, e₄ et e₅ de la figure 5a générées respectivement à des instants tce₁, tce₂, tce₃, tce₄ et tce₅. Chacune des commandes comporte une information relative au niveau de gris auquel le pixel doit être illuminé, notée sur la figure g(teᵢ), avec i = {1, 2, 3, 4, 5}.

Outre les commandes d'affichage Ceᵢ générées sur détection d'un évènement eᵢ, une commande d'affichage de rafraîchissement Cr,e₂ est générée à l'instant t'e₂+Δ*tₚ* suite à la non-détection d'un nouvel évènement consécutif à celle de l'événement e₂ pendant une durée Δ*tₚ*.

La commande d'affichage de rafraichissement Cr,e₂ pourra être générée avec les mêmes caractéristiques d'affichage, et par exemple le même niveau de gris g(te₂), que celles comprises dans la commande d'affichage sur évènement Ce₂. En variante, la commande d'affichage de rafraichissement Cr,e₂ pourra être générée avec des caractéristiques d'affichage déterminées en fonction de celles déterminées pour la commande d'affichage sur évènement Ce₂.

Dans un mode particulier de réalisation du procédé proposé, la gestion de l'intervalle de temps de rafraîchissement pour chaque pixel peut être réalisée à l'aide d'un temporisateur, réglé à la valeur Δ*tₚ* définie pour le pixel et déclenché à chaque génération de commande d'affichage pour le pixel. En variante, un temporisateur de rafraîchissement pourra être enclenché pour un pixel lors de chaque transmission d'un signal de commande d'affichage pour le pixel. Ce temporisateur pourra être stoppé sur détection d'un nouvel évènement pour lequel le système de commande d'affichage détermine qu'une commande d'affichage doit être générée, et sur expiration lorsqu'aucune commande d'affichage n'a été générée durant l'intervalle Δ*tₚ* ce qui donnera lieu à la génération d'une commande d'affichage de rafraîchissement pour le pixel.

En variante, le rafraîchissement des pixels de la matrice peut être effectué en groupe, et il peut être procédé à un rafraichissement systématique et périodique de pixels de la matrice à une fréquence de rafraîchissement prédéterminée. L'avantage de ce mode de réalisation est d'éviter une gestion individualisée de chaque pixel pour ce qui concerne le rafraichissement. Le rafraichissement de la matrice est alors répété à une cadence au moins égale à l'intervalle de rafraichissement le plus court défini pour un pixel.

Dans ce mode de réalisation du procédé proposé, le rafraichissement de la matrice de pixels est effectué périodiquement, tout en restant complètement décorrélé et indépendant de la séquence de commandes d'affichage sur évènements. La génération de la commande de rafraichissement d'affichage est indépendante de celle de la commande d'affichage sur événement, et la fréquence de rafraichissement d'affichage est choisie de manière à ce que l'écart entre un affichage sur évènement de chaque pixel et l'affichage de rafraichissement immédiatement subséquent n'excède pas un intervalle de temps Δ*tₚ* qui, comme discuté ci-dessus, pourra être choisi en fonction de la persistance rétinienne de l'œil humain de l'ordre de 40ms à 150ms. Ce mode « mixte » d'affichage permet de combiner un affichage sur évènement fonctionnant de manière asynchrone avec un affichage périodique de rafraichissement effectué de manière synchrone. Ainsi, en fonction de la survenance des évènements pour chaque pixel, l'affichage de rafraichissement du pixel pourra survenir dans un très court laps de temps suivant un affichage sur évènement du pixel, ou bien à l'issue d'une durée suivant un affichage sur évènement du pixel définie comme l'écart maximum entre deux affichages du pixel. Ce mode mixte permet d'alléger la gestion du rafraichissement de l'affichage, tout en limitant grâce à l'affichage sur évènement asynchrone pour chaque pixel l'affichage de l'information de redondance, étant donné que l'affichage asynchrone permet de choisir des fréquences de rafraichissement faibles en comparaison des systèmes actuels.

En référence à la figure 1, chaque génération de commande d'affichage sur évènement pourra par exemple donner lieu à la mise en mémoire 103 par l'unité de traitement de données 102 de caractéristiques d'affichage relatives à la commande générée, de sorte que ces caractéristiques puissent être récupérées par l'unité 102 pour la gestion de l'affichage de rafraichissement.

La figure 5d représente schématiquement une suite de commandes d'affichage générées selon un autre mode de réalisation du procédé proposé appliqué à la séquence d'évènements des figures 5a et 5b.

En référence à la figure 5b, l'événement e4 est détecté avec un espacement temporel te₄ - te₃ inférieur à l'intervalle de temps Δ*ₜₘᵢₙ* défini comme l'intervalle de temps minimum séparant deux commandes d'affichage sur évènement successives du pixel.

La séquence de commandes d'affichage de la figure 5d diffère de la séquence illustrée par la figure 5c en ce qu'aucune commande d'affichage n'est générée pour l'événement e₄, étant donné que l'écart temporel entre l'instant de détection de cet évènement et l'instant de détection de l'événement immédiatement précédent est inférieur à un seuil prédéfini *Δₜₘᵢₙ*.

Dans un mode particulier de réalisation du procédé proposé, la gestion de l'intervalle de temps de séparation pour chaque pixel peut être réalisée à l'aide d'un temporisateur, réglé à la valeur Δ*ₜₘᵢₙ* définie pour le pixel et déclenché à chaque génération de commande d'affichage sur évènement pour le pixel. Par exemple, un évènement détecté alors que ce temporisateur n'a pas expiré pour le pixel pourra conduire à ignorer l'événement et ne pas générer de commande d'affichage sur évènement correspondante.

Les caractéristiques d'affichage portées par l'information asynchrone pour cet évènement ignoré pourront être aussi ignorées. En variante, on pourra prévoir une mise en œuvre selon laquelle, quand bien même aucune commande d'affichage sur évènement n'est générée, les caractéristiques d'affichage correspondant à l'évènement sont consignées en mémoire 103 pour être utilisées ultérieurement, par exemple pour le prochain affichage de rafraichissement du pixel.

Selon un mode de réalisation, un seul temporisateur peut être utilisé pour la gestion de l'intervalle de rafraichissement et celle de l'intervalle de séparation. Ce temporisateur peut être déclenché à chaque génération de commande d'affichage pour le pixel, en consignant en mémoire un indicateur permettant de distinguer les commandes d'affichage sur évènement des commandes d'affichage de rafraichissement.

La figure 6 illustre différentes périodes de la gestion de l'affichage d'un pixel selon un mode de réalisation du procédé proposé, indiquées sur un axe des temps.

La détection d'un évènement eᵢ à partir duquel il est généré une commande d'affichage sur évènement constitue le point de départ noté teᵢ d'une période dite période réfractaire, durant laquelle il n'est pas considéré utile d'afficher un nouvel événement. Cette période a une durée notée Δ*ₜₘᵢₙ* qui correspond à l'intervalle de séparation décrit ci-dessus, et peut être choisie dans un intervalle allant de 1ms à 40ms, et fixée par exemple à une valeur de l'ordre de 30 ms. La valeur de cette période pourra être choisie en fonction des limitations de calcul du système implémentant le procédé proposé et/ou le degré de précision souhaité. Elle pourra en outre être définie de manière dynamique par le type de mouvements potentiellement présents dans la scène.

Au-delà de cette période Δ*ₜₘᵢₙ*, il est préférable d'afficher un nouvel évènement détecté pour le pixel. Deux instants notés respectivement tp₁ et tp₂ définissent un intervalle temporel pendant lequel il est préférable d'effectuer l'affichage de rafraichissement du pixel. L'écart temporel entre teᵢ et tp₂ correspond à l'intervalle de rafraichissement, noté Δ*tₚ*, décrit ci-dessus. Le système est configuré pour générer un affichage de rafraichissement du dernier affichage sur évènement du pixel pendant l'intervalle de temps Δ*tₚ*. En variante, le point de départ teᵢ de l'intervalle Δ*tₚ* peut par exemple correspondre à la génération de la commande d'affichage sur événement, ou bien aussi à la transmission de la commande une fois générée.

Le seuil déterminé par tp₁ peut être utilisé pour éviter d'effectuer un affichage de rafraichissement du pixel qui soit trop proche de l'affichage sur évènement initial.

Selon un mode de réalisation, un évènement eᵢ₊₁ consécutif à l'évènement ei détecté comme survenant après la période réfractaire de durée Δ*ₜₘᵢₙ*, conduit à la génération d'une commande d'affichage. Le ou les temporisateurs de gestion de la période réfractaire et de gestion de l'affichage de rafraichissement, respectivement, sont réinitialisés à partir de l'instant teᵢ₊₁, qui peut correspondre à l'instant de détection de l'évènement eᵢ₊₁, à l'instant de génération de la commande correspondante ou de la transmission de celle-ci.

Par contre, si aucun évènement eᵢ₊₁ consécutif à l'évènement eᵢ n'est détecté à l'instant tp₁, une commande d'affichage de rafraichissement est générée dans l'intervalle de temps [tp₁, tp₂], par exemple à l'instant tp₁, ou bien à tout instant entre tp₁ et tp₂, y compris tp₂, si à cet instant aucun évènement eᵢ₊₁ consécutif à l'évènement eᵢ n'a encore été détecté.

On peut comparer les modes d'affichage synchrone et asynchrone en s'intéressant au taux de pixels rafraîchis à une période donnée. Par exemple, un affichage du type conventionnel (dit « synchrone ») sur un écran avec une fréquence de rafraîchissement de 200 Hz donnera lieu à un affichage d'un pixel donné toutes les 5 ms. Un affichage de type asynchrone pourra être effectué avec un écart minimum de 5 ms et un écart maximum de 40 ms, seuls les pixels correspondant à une zone de mouvement dans la séquence d'images affichée étant affichés toutes les 5 ms, les affichages de rafraîchissement n'intervenant par exemple que toutes les 40 ms pour les pixels ne correspondant pas à une zone de mouvement.

Afin de connaître le gain en termes de pixels non rafraîchis du mode d'affichage asynchrone, on peut déterminer un nombre de pixels appartenant à des zones en mouvements, puis calculer le pourcentage de pixels non rafraîchis à 200 Hz en mode asynchrone par rapport au mode synchrone sur une durée d'une seconde.

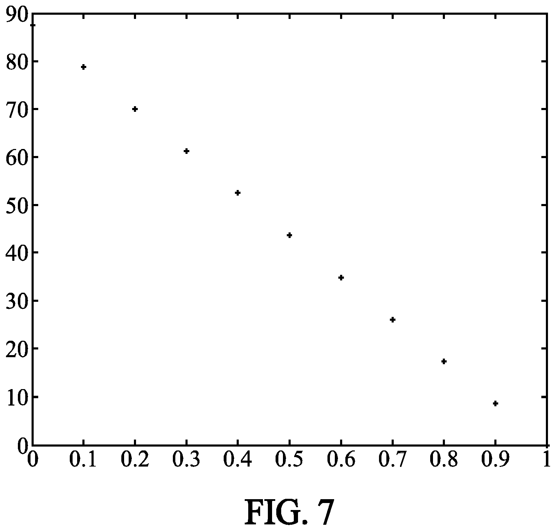

La figure 7 montre la variation du nombre de pixels non rafraîchis en mode asynchrone par rapport à un affichage synchrone en fonction du nombre de pixels contenus dans les zones de mouvement. Les quantités sont exprimées en pourcentages, et le graphe de la figure 7 montre le pourcentage de pixels non rafraîchis (en ordonnée) en asynchrone par rapport à un mode synchrone pour un afficheur à 200 Hz en fonction du pourcentage de pixels appartenant à une zone de mouvement (en abscisse).

Bien que décrits à travers un certain nombre d'exemples de réalisation détaillés, le procédé de commande d'affichage et l'équipement pour la mise en œuvre du procédé comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

Par exemple, dans un mode de réalisation, le procédé proposé est un procédé de commande d'affichage d'images animées sous forme d'une matrice de pixels, comprenant : recevoir de l'information asynchrone indiquant, pour chaque pixel de la matrice, des événements successifs dépendant de variations de lumière dans une scène; activer les pixels de la matrice à des instants respectifs, chaque instant d'activation d'un pixel étant déterminé par un événement respectif indiqué par l'information asynchrone pour ledit pixel ; après activation d'un pixel de la matrice à un instant déterminé par un événement indiqué par l'information asynchrone, répéter l'activation dudit pixel sensiblement au même niveau d'activation à des instants définis par une séquence de rafraichissement jusqu'à détermination d'un prochain instant d'activation dudit pixel par les événements indiqués par l'information asynchrone.

En variante, on peut activer les pixels de la matrice à des instants respectifs déterminés par des événements indiqués par l'information asynchrone pour lesdits pixels.

Il est en outre proposé un dispositif correspondant de commande d'affichage d'images animées sous forme d'une matrice de pixels, comprenant : une entrée pour recevoir de l'information asynchrone indiquant, pour chaque pixel de la matrice, des événements successifs dépendant de variations de lumière dans une scène; une unité d'activation des pixels de la matrice, agencée pour activer chaque pixel de la matrice à des instants déterminés par des événements respectifs indiqués par l'information asynchrone pour ledit pixel ; et après activation d'un pixel de la matrice à un instant déterminé par un événement indiqué par l'information asynchrone, répéter l'activation dudit pixel sensiblement au même niveau d'activation à des instants définis par une séquence de rafraichissement jusqu'à détermination d'un prochain instant d'activation dudit pixel par les événements indiqués par l'information asynchrone.

De plus, différents aspects et caractéristiques décrits ci-dessus peuvent être mis en œuvre ensemble, ou séparément, ou bien substitués les uns aux autres, et l'ensemble des différentes combinaisons et sous combinaisons des aspects et caractéristiques font partie de la portée de l'invention. En outre, il se peut que certains systèmes et équipements décrits ci-dessus n'incorporent pas la totalité des modules et fonctions décrits pour les modes de réalisation préférés.

Les informations et signaux décrits dans le présent document peuvent être représentés selon une multitude de technologies et de techniques. Par exemple, les instructions, messages, données, commandes, informations, signaux, bits et symboles peuvent être représentés par des tensions, intensités, ondes électromagnétiques ou une combinaison de ces derniers.

En fonction du mode de réalisation choisi, certains actes, actions, évènements ou fonctions de chacune des méthodes décrites dans le présent document peuvent être effectués ou se produire selon un ordre différent de celui dans lequel ils ont été décrits, ou peuvent être ajoutés, fusionnés ou bien ne pas être effectués ou ne pas se produire, selon le cas. En outre, dans certains modes de réalisation, certains actes, actions ou évènements sont effectués ou se produisent concurremment et non pas successivement.

## Revendications

1. Procédé de commande pour l'affichage d'images sous forme d'une matrice de pixels, le procédé comprenant, pour chaque pixel d'un ensemble de pixels de la matrice :
recevoir (500), depuis un capteur de vision asynchrone basé sur évènements (201), de l'information asynchrone représentant des évènements pour le pixel, chaque événement parmi lesdits événements correspondant à une variation respective d'intensité lumineuse au-delà d'un seuil sur ledit pixel,
chaque événement parmi lesdits événements étant associé à au moins une information lumineuse respective et à un instant de survenance respectif de ladite variation respective d'intensité lumineuse au-delà du seuil ;
le procédé comprenant en outre :
identifier un premier événement pour ledit pixel parmi lesdits événements représentés par l'information asynchrone reçue ;
déterminer l'instant de survenance respectivement associé audit premier événement ;
déterminer, à partir de l'instant de survenance déterminé, un instant d'activation dudit pixel ;
commander (501) une première activation du pixel à l'instant d'activation déterminé ;
commander (502) au moins une deuxième activation du pixel pour répéter la première activation du pixel à des instants respectifs définis par une séquence de rafraichissement.

2. Procédé selon la revendication 1, dans lequel la commande de première activation du pixel est répétée selon la séquence de rafraîchissement jusqu'à détermination d'un prochain instant d'activation du pixel par les évènements de l'information asynchrone.

3. Procédé selon la revendication 1, dans lequel la séquence de rafraîchissement définit des instants d'activation du pixel séparés par un intervalle de temps.

4. Procédé selon la revendication 3, dans lequel l'intervalle de temps est déterminé en fonction de la persistance rétinienne de l'œil humain.

5. Procédé selon les revendications 3 ou 4, dans lequel l'intervalle de temps est supérieur à 40 ms et inférieur à 800 ms.

6. Procédé selon la revendication 3, dans lequel le rafraichissement de l'activation est effectué simultanément pour une pluralité de pixels de la matrice de pixels.

7. Procédé selon l'une quelconque des revendications 1 à 6, dans lequel la réception de l'information lumineuse comprend un train d'impulsions binaires positionnées dans le temps en fonction des variations de lumière dans la scène pour le pixel.

8. Procédé selon l'une quelconque des revendications 1 à 6, dans lequel l'information représentant le premier évènement comprend une première impulsion positionnée dans le temps en fonction des variations de lumière dans la scène pour le pixel, et une deuxième impulsion positionnée dans le temps en fonction d'un niveau de gris pour l'activation du pixel.

9. Procédé selon l'une quelconque des revendications précédentes, comprenant en outre :
- détecter dans l'information asynchrone une première information représentant le premier événement ;
- détecter dans l'information asynchrone une deuxième information représentant un deuxième évènement pour le pixel ;
- lorsque l'écart temporel entre les premier et deuxième évènements est inférieur à un seuil, ne pas commander l'activation du pixel à un instant d'activation déterminé par le deuxième événement.

10. Procédé selon la revendication 9. dans lequel le seuil est compris entre 1ms et 40ms.

11. Dispositif de commande pour l'affichage d'images sous forme d'une matrice de pixels, comprenant :
• une interface d'entrée configurée pour recevoir de l'information asynchrone représentant des évènements pour au moins un pixel de la matrice ;
• une unité de commande d'activation de pixel, couplée de manière opérationnelle à l'interface d'entrée, et configurée pour commander l'activation du pixel selon le procédé de l'une quelconque des revendications 1 à 10.

12. Dispositif de commande selon la revendication 11, comprenant en outre une interface de sortie, couplée de manière opérationnelle à l'unité de commande d'activation de pixel, pour délivrer des commandes d'activation sous la forme de stimulations électriques appliquées à une prothèse visuelle d'un dispositif d'aide à la vision.

13. Afficheur d'images, comprenant :
- une unité d'affichage procurant une matrice de pixels ; et
- un dispositif selon l'une des revendications 11 à 12 pour commander l'unité d'affichage.

14. Produit programme d'ordinateur, chargeable dans une mémoire associée à un dispositif selon les revendications 11 à 13, et comprenant des portions de code pour la mise en œuvre des étapes d'un procédé selon l'une quelconque des revendications 1 à 10 lors de l'exécution dudit programme par le dispositif.

## Patentansprüche

1. Steuerungsverfahren für die Anzeige von Bildern in Form einer Matrix von Pixeln, wobei das Verfahren für jedes Pixel einer Gesamtheit von Pixeln der Matrix aufweist:
Empfangen (500) von asynchroner Information aus einem auf Ereignissen basierenden asynchronen Sehsensor, wobei die asynchrone Information Ereignisse für das Pixel repräsentiert und jedes der Ereignisse einer eine Schwelle überschreitenden jeweiligen Lichtintensitätsänderung an dem Pixel entspricht,
wobei jedes der Ereignisse mit mindestens einer jeweiligen Lichtinformation und mit einem jeweiligen Erscheinungszeitpunkt der die Schwelle überschreitenden jeweiligen Lichtintensitätsänderung verknüpft ist;
wobei das Verfahren ferner aufweist:
Identifizieren eines ersten Ereignisses für das Pixel aus den von der empfangenen asynchronen Information repräsentierten Ereignissen;
Bestimmen des Erscheinungszeitpunkts, der jeweils mit dem ersten Ereignis verknüpft ist;
Bestimmen eines Aktivierungszeitpunkts des Pixels ausgehend von dem bestimmten Erscheinungszeitpunkt;
Steuern (501) einer ersten Aktivierung des Pixels an dem bestimmten Aktivierungszeitpunkt;
Steuern (502) mindestens einer zweiten Aktivierung des Pixels, um die erste Aktivierung des Pixels an jeweiligen Zeitpunkten, die durch eine Auffrischungssequenz definiert sind, zu wiederholen.

2. Verfahren nach Anspruch 1, in welchem das Steuern der ersten Aktivierung des Pixels gemäß der Auffrischungssequenz wiederholt wird bis zu einer Bestimmung eines nächsten Aktivierungszeitpunkts des Pixels durch die Ereignisse der asynchronen Information.

3. Verfahren nach Anspruch 1, in welchem die Auffrischungssequenz Aktivierungszeitpunkte des Pixels, die durch ein Zeitintervall getrennt sind, definiert.

4. Verfahren nach Anspruch 3, in welchem das Zeitintervall in Abhängigkeit von dem Netzhautnachleuchten des menschlichen Auges bestimmt wird.

5. Verfahren nach Anspruch 3 oder 4, in welchem das Zeitintervall größer als 40ms und kleiner als 800ms ist.

6. Verfahren nach Anspruch 3, in welchem das Auffrischen der Aktivierung für mehrere Pixel der Pixelmatrix gleichzeitig erfolgt.

7. Verfahren nach einem der Ansprüche 1 bis 6, in welchem der Empfang der Lichtinformation eine Folge von binären Pulsen aufweist, die in Abhängigkeit der Lichtänderungen in der Szene für das Pixel zeitlich angeordnet sind.

8. Verfahren nach einem der Ansprüche 1 bis 6, in welchem die Information, die das erste Ereignis repräsentiert, einen ersten Puls, der in Abhängigkeit von den Lichtänderungen in der Szene für das Pixel zeitlich angeordnet ist, und einen zweiten Puls, der in Abhängigkeit von einem Graupegel für die Aktivierung des Pixels zeitlich angeordnet ist, aufweist.

9. Verfahren nach einem der vorstehenden Ansprüche, ferner aufweisend:
- Detektieren einer ersten Information, die das erste Ereignis repräsentiert, in der asynchronen Information;
- Detektieren einer zweiten Information, die ein zweites Ereignis für das Pixel repräsentiert, in der asynchronen Information;
- wenn der Zeitabstand zwischen dem ersten und zweiten Ereignis kleiner als eine Schwelle ist, Unterlassen der Steuerung der Aktivierung des Pixels an einem von dem zweiten Ereignis bestimmten Aktivierungszeitpunkt.

10. Verfahren nach Anspruch 9, in welchem die Schwelle im Bereich zwischen 1ms und 40ms ist.

11. Steuerungsvorrichtung für die Anzeige von Bildern in Form einer Matrix von Pixeln, aufweisend:
* eine Eingabeschnittstelle, die konfiguriert ist, asynchrone Information zu empfangen, die Ereignisse für mindestens ein Pixel der Matrix repräsentiert;
* eine Pixelaktivierungssteuerungseinheit, die mit der Eingabeschnittstelle operativ gekoppelt ist und konfiguriert ist, die Aktivierung des Pixels gemäß dem Verfahren nach einem der Ansprüche 1 bis 10 zu steuern.

12. Steuerungsvorrichtung nach Anspruch 11, ferner aufweisend eine Ausgabeschnittstelle, die mit der Pixelaktivierungssteuerungseinheit operativ gekoppelt ist, um Aktivierungsbefehle in Form von elektrischen Anregungen, die an eine Sehprothese einer Sehhilfevorrichtung angelegt sind, auszugeben.

13. Bildanzeiger, aufweisend:
- eine Anzeigeeinheit, die eine Matrix von Pixeln bereitstellt; und
- eine Vorrichtung nach einem der Ansprüche 11 bis 12 zur Steuerung der Anzeigeeinheit.

14. Computerprogrammprodukt, das in einen Speicher, der mit einer Vorrichtung nach den Ansprüchen 11 bis 13 verbunden ist, geladen werden kann und Codeabschnitte aufweist, um die Schritte eines Verfahrens nach einem der Ansprüche 1 bis 10 durchzuführen, wenn das Programm von der Vorrichtung zur Ausführung gebracht wird.

## Claims

1. Method for controlling a display of images as a pixel array, the method comprising, for each pixel of a set of pixels of the array:
receiving (500), from an event-based asynchronous vision sensor (201), asynchronous information representing events concerning the pixel, each event among said events corresponding to a respective light intensity variation beyond a threshold on said pixel,
each event among said events being associated with at least one respective light information and with a respective occurrence time of said light intensity variation beyond the threshold;
the method further comprising:
identifying a first event for said pixel among said events represented by the received asynchronous information;
determining the occurrence time respectively associated with said first event;
determining, based on the determined occurrence time, an activation time for said pixel;
actuating (501) a first activation of the pixel at the determined activation time;
actuating (502) at least a second activation of the pixel in order to repeat the first activation of the pixel at respective times defined by a refresh sequence.

2. Method according to claim 1, wherein the first activation actuation of the pixel is repeated according to the refresh sequence until determination of a next activation time of the pixel by events of the asynchronous information.

3. Method according to claim 1, wherein the refresh sequence defines activation times of the pixel separated by a time interval.

4. Method according to claim 3, wherein the time interval is determined according to retinal persistence of human eye.

5. Method according to claim 3 or 4, wherein the time interval is greater than 40 ms and lower than 800 ms.

6. Method according to claim 3, wherein the activation is refreshed simultaneously for a plurality of pixels of the pixel array.

7. Method according to any of claims 1 to 6, wherein the reception of light information comprises a train of binary pulses positioned over time as a function of light variations in the scene for the pixel.

8. Method according to any of claims 1 to 6, wherein the information representing the first event comprises a first pulse positioned over time as a function of light variations in the scene for the pixel, and a second pulse positioned over time as a function of a greyscale for the activation of the pixel.

9. Method according to any of the previous claims, further comprising:
- detecting in the asynchronous information a first item of information representing the first event;
- detecting in the asynchronous information a second item of information representing a second event for the pixel;
- when a time gap between the first and second events is lower than a threshold, not actuating the activation of the pixel at an activation time determined by the second event.

10. Method according to claim 9, wherein the threshold is comprised between 1 ms and 40 ms.

11. Device for controlling the display of images as a pixel array, comprising:
• an input interface configured to receive asynchronous information representing events for at least one pixel of the array;
• a pixel activation control unit, coupled operationally with the input interface, and configured to actuate activation of the pixel according to a method of any of claims 1 to 10.

12. Control device according to claim 11, further comprising an output interface, coupled operationally with the pixel activation control unit, to deliver activation commands in the form of electric stimulations applied to a visual prosthesis of a vision aid device.

13. Image display, comprising:
- a display unit providing a pixel array; and
- a device according to any of claims 11 to 12 for controlling the display unit.

14. Computer program product, loadable in a memory associated with a device according to any of claims 11 to 13, and comprising code portions for carrying out steps of a method according to any of claims 1 to 10 when said program is executed by the device.
